# EUROPEAN PATENT SPECIFICATION

(11) **EP 4 069 849 B1**
(45) Date of publication and mention of the grant of the patent: **22.01.2025**
(21) Application number: 20907151.3
(22) Date of filing: 05.05.2020
(51) Int. Cl.: C12N 15/113, C12N 15/85, C12N 5/10

(54) **LIVER-SPECIFIC PROMOTER AND APPLICATION THEREOF**
LEBERSPEZIFISCHER PROMOTOR UND VERWENDUNG DAVON
PROMOTEUR SPÉCIFIQUE DU FOIE ET SON APPLICATION

(30) Priority: 25.12.2019 CN 201911357788
(43) Date of publication of application: 12.10.2022
(73) Proprietor: Jinfan Biomedical Technology (Wuhan) Co., Ltd., Wuhan, Hubei 430000 (CN)
(72) Inventor: LIU, Guangmeng, Wuhan, Hubei 430000 (CN); PAN, Xing, Wuhan, Hubei 430000 (CN); ZHANG, Sheng, Wuhan, Hubei 430000 (CN); FANG, Wenjing, Wuhan, Hubei 430000 (CN); HE, Xiaobin, Wuhan, Hubei 430000 (CN)
(74) Representative: Bayramoglu et al.
(86) International application number: PCT/CN2020/088604
(87) International publication number: WO 2021/128692

(56) References cited:
- WO-A1-2016/146757
- CN-A- 107 427 557
- CN-A- 107 864 657
- CN-A- 109 562 191
- WANG ZHONGYAN ET AL: "Modulation of hepatocyte nuclear factor-4[alpha] function by the peroxisome-proliferator-activated receptor-[gamma] co-activator-1[alpha] in the acute-phase response", BIOCHEMICAL JOURNAL, vol. 415, no. 2, 15 October 2008 (2008-10-15), GB, pages 289 - 296, XP093051727, ISSN: 0264-6021, Retrieved from the Internet <URL:https://www.ncbi.nlm.nih.gov/pmc/articles/PMC3552497/pdf/nihms-435096.pdf> DOI: 10.1042/BJ20080355
- SHEN RONG-FONG: "Nucleic Acids Research Tissue-specific expression of the human cil-antitrypsin gene is controlled by multiple cis- regulatory elements", NUCLEIC ACIDS RESEARCH, vol. 15, no. 20, 1 January 1987 (1987-01-01), pages 8399 - 8415, XP093051729, Retrieved from the Internet <URL:https://www.ncbi.nlm.nih.gov/pmc/articles/PMC306367/pdf/nar00264-0277.pdf>

## Description

### Technical Field

The present disclosure belongs to the field of gene therapy, and more specifically relates to a liver-specific promoter and application thereof.

### Background

Gene therapy refers to introducing exogenous normal genes into target cells, to correct or compensate a disease caused by gene defect or abnormality, finally achieving the treatment goal. Since the first gene therapy regimen for human entered into clinical trial in 1989, it has been developed for more than 30 years. Although the process was quite rough, with the optimization of the vector virus and delivery mechanism, in recent years, the gene therapy has been developed rapidly, showing a good prospect in terms of therapies of cancer and rare diseases. Only in 2017, the gene therapy made multiple milestone breakthroughs, U.S. Food and Drug Administration (FDA) successively approved two CAR-T therapies (Kymriah and Yescarta) and first gene therapy for "targeting genetic eye disease mutation" (Luxturna), so this year could be called a year of gene therapy being "rising abruptly based on accumulated efforts".

Adeno-associated virus (AAV) belongs to the Parvoviridae family, and is a class of virus having small virion, replication defect, and no envelop, so far no wild type AAV has been found to cause human disease. Artificially modified recombinant AAV (rAAV) has advantages such as good safety, low immunogenicity, broad tissue tropism, and no integration into the genome of a host cell, in recent years, using rAAV as a gene therapy vector has become a hotspot in the gene therapy study. As early as 2012, European State food and drug administration approved the first gene therapy drug, a rAAV vector-based lipoprotein lipase gene therapy drug (Glybera); on December 19, 2017, U.S. Food and Drug Administration approved the first rare diseases gene therapy drug (Luxturna) for treating congenital amaurosis (caused by RPE65 gene mutation), this drug also uses rAAV as the gene therapy vector; on May 24, 2019, U.S. FDA also approved a rare disease gene therapy drug (Zolgensma) of Novartis for treating severe muscular atrophy type 1 (SMA I). By November 2019, gene therapy clinical trial protocols using rAAV as the vector being registered on ClinicalTrials.gov are up to more than 200 (ClinicalTrials.gov).

Using the recombinant AAV as the gene therapy vector also has obvious shortcomings, namely its capacity for loading exogenous gene is small, the upper limit for loading exogenous gene by single-stranded genome AAV is 4.7 kb, length for loading exogenous gene further increases, consequently the integrity of virion packing decreases. Due to restriction by the loading capacity, some larger genes are not suited to be delivered by the rAAV vector, such as Hemophilia A pathogenic gene blood coagulation factor VIII, the size of its cDNA is 7056bp, it encodes FVIII precursor protein of 235 1aa, this far exceeds the loading capacity of rAAV.

Hemophilia is a class of hereditary blood coagulation dysfunction disease caused by blood coagulation factor deficiency, its common type is hemophilia A (HA) and hemophilia B (HB). By estimation on the basis of incidence of hemophilia being 5-10/100 thousand, it is expected that the number of hemophilia patients in China at present is approximately 100 thousand, and approximately one million in the world.

HA results from blood coagulation factor VIII deficiency or functional deficiency caused by FVIII (F8) gene mutation on chromosome X, it accounts for about 80% of whole hemophilia. HB manifests as blood coagulation factor IX (FIX) deficiency, it accounts for about 20% of whole hemophilia patients, and it is caused by FIX (F9) gene mutation on chromosome X.

At present, the main method for treating hemophilia is replacement therapy. The replacement therapy is carried out by infusion of exogenous recombinant blood coagulation factors to relieve its hemorrhagic complication, and to prevent loss of the function. However, because half-life of the blood coagulation factor VIII is short (8-12 hours), it is required to inject 2-3 times every week, the frequent treatments severely impact the life quality of the patients. The gene therapy provides the therapies of hemophilia as well as other some single gene inheritance diseases with a new pathway. Hemophilia gene therapy is to introduce exogenous gene normally encoding blood coagulation factors into the patient's body, and to express and secret a therapeutic level of blood coagulation factors within the cells, thereby achieving the goal of completely curing hemophilia. Blood coagulation factor VIII is not required to be supplemented to a normal level to function, it is only required to restore to 5% of the normal level to achieve the effect of hemorrhage prevention, making hemophilia A an ideal gene therapy indication.

According to the features such as large blood coagulation factor VIII gene and restriction to AAV vector loaded gene by capacity, Lind P. et al. replaced B domain of the blood coagulation factor VIII (760-1667aa) with a linker peptide of 14 amino acids (an SQ sequence), and reduced the size of this gene by 40% to 4.4kb (Lind P et al., Eur. J. Biochem. 232:19-27,1995), this B domain deleted FVIII (BDD-FVIII) is a commonly used revision at present for FVIII protein expression; Jenny McIntosh et al. enhanced in vivo expression of this protein by inserting 17aa short chain polypeptide V3 (V3-FVIII) including six glycosylation sites into the SQ sequence (Jenny McIntosh et al., BLOOD. 121(17), 2013). However, whether BDD-FVIII or V3-FVIII, its size approaches the upper limit of loading of rAAV, leaving a space of only approximately 300bp to the regulatory element (promoter, Poly A) for regulatory target gene expression.

The main synthesis site of FVIII are liver cells and hepatic sinusoidal endothelial cells, at present, the strategy for treating hemophilia A by using rAAV gene is to reduce the target gene FVIII and meanwhile to assemble a promoter, BDD-FVIII or V3-FVIII, polyA onto the limited space of the same AAV vector by a liver-specific promoter of small size, e.g., an expression cluster of BMN270 of BIOMARIN company is HLP-BDD FVIII-sPA (ClinicalTrials.gov number, NCT02576795), and an expression cluster of SPK-8011 of SPARK company is TTRm-BDD FVIII-Rabbit beta globin poly A (ClinicalTrials.gov number, NCT03003533), both drive expression of target gene by using a liver-specific promoter of small size, phase I/II clinical trials have achieved certain effects (Rangarajan S et al., The New England Journal of Medicine. 2017; Lindsey A.G et al., Blood. 130:604, 2017), wherein the marketing application of the hemophilia A gene therapy drug BMN270 of BIOMARIN has been submitted to EMA. However, due to the constraint by the promoter size, the difference between its initiating intensity and that of some promoters with larger size is not small.

An efficient promoter can continuously and intensively initiate expression of a target gene, reduce the dose required to achieve the therapeutic effect, and reduce frequency of administration, thereby greatly reducing the treatment cost and body's immunological reaction. However, due to the restriction of rAAV loading capacity, when a larger gene such as blood coagulation factor VIII is loaded, it is bound to reduce the length of the promoter, correspondingly the key expression regulatory element included in a deletion sequence is lost, and expression intensity and specificity of the target gene reduce as well. How to maintain the intensity and the specificity of initiating the target gene by the promoter in case of reducing the size of the promoter to the greatest extent, is an important issue to be solved when a larger gene is delivered by using rAAV as the vector.

### Summary

For the above-mentioned shortcomings or improvement requirements in the prior art, the present disclosure provides a liver-specific promoter and application thereof, and aims to construct a strong promoter with a small size and to apply the promoter in driving expression of a larger exogenous gene (such as FVIII blood coagulation factor gene), thereby solving the technical problem of limited loading capacity of the rAAV vector used in the existing gene therapy.

To achieve the above-mentioned purpose, according to one aspect of the present disclosure, a nucleic acid molecule is provided, including:
#
   (a) a first polynucleotide having the nucleotide sequence shown in SEQ ID NO: 1 or SEQ ID NO: 2; and
   (b) a second polynucleotide having the nucleotide sequence shown in SEQ ID NO: 3;
wherein (b) and (a) are 5'-3' linked, and the nucleic acid molecule is able to facilitate transcription of the heterogeneous polynucleotide in liver tissue of mammal.

Preferably, the nucleic acid molecule includes:
(a) a first polynucleotide having the nucleotide sequence shown in SEQ ID NO: 1 or SEQ ID NO: 2; and
(b) a second polynucleotide having the nucleotide sequence shown in SEQ ID NO: 3; and
(c) a third polynucleotide having the nucleotide sequence shown in SEQ ID NO: 4;
wherein (c), (b) and (a) are 5'-3' linked, and the nucleic acid molecule is able to facilitate the transcription of the heterogeneous polynucleotide in liver tissue of mammal.

According to another aspect of the present disclosure, an application of the nucleic acid molecule is provided, and the nucleic acid molecule is used as a liver-specific promoter.

According to another aspect of the present disclosure, an expression vector is provided, wherein the expression vector includes the nucleic acid molecule.

Preferably, the expression vector is a plasmid or a viral vector.

Preferably, the expression vector is rAAV.

According to another aspect of the present disclosure, a mammal host cell is provided, including the expression vector.

In general, by means of the above technical solution conceived by the present disclosure, compared to the prior art, the following beneficial effects can be achieved:
The present disclosure aims to construct a strong promoter of small size and apply the promoter into the rAAV gene therapy of hemophilia A. The present disclosure provides a small-size recombinant nucleic acid sequence for regulating high specific expression of genes in a liver system. Compared with the currently reported other sequences of similar size, the recombinant regulatory sequence fragment has the advantage that the ability of driving expression of reporter gene and human coagulation factor FVIII in the liver system is significantly enhanced, thereby being suitable for recombinant adeno-associated virus (rAAV)-mediated gene therapy.

### Brief Description of the Drawings

FIG. 1 shows a structural schematic diagram of PFD-rAAV-CMV-mCHERRY-bGHpA vector.
FIG. 2 shows a structural schematic diagram of PFD-rAAV-HLP-BDD-FVIIIopt(WJ)- spolyA vector.
FIG. 3 shows a comparison of effects of different promoters for initiating mCHERRY fluorescence in mouse liver tissue.
FIG. 4 shows a comparison of effects of different promoters for initiating mCHERRY fluorescence in mouse liver tissue.
FIG. 5 shows a gray scale scan diagram of effects of different promoters for initiating mCHERRY fluorescence in mouse liver tissue.
FIG. 6 shows SDS-PAGE protein gel silver staining diagrams of different rAAV viruses.
FIG. 7 shows different AAV virus titers determined by an RT-PCR method.
FIG. 8 shows a comparison of effects of different promoters for expressing FVIII in C57 mice.

### Detailed Description of the Embodiments

In order to make the purpose, the technical solution and the advantage of the present disclosure more clear and distinct, the present disclosure will be described in more detail below in conjunction with the accompanying drawings and the examples. It should be understood that, the specific examples described herein are merely intended to explain the present disclosure, and not to limit the present disclosure. In addition, the technical features involved in the various embodiments of the present disclosure described below can be combined with one another, as long as they do not conflict with one another.

The present disclosure describes a nucleic acid molecule, including:
(a) a first polynucleotide having a nucleotide sequence shown in SEQ ID NO: 1 or SEQ ID NO: 2; and
(b) a second polynucleotide having a nucleotide sequence shown in SEQ ID NO: 3;
wherein (b) and (a) are 5'-3' linked, and the nucleic acid molecule is able to facilitate efficient transcription of a heterogeneous polynucleotide in liver tissue of mammal.

In some examples, the nucleic acid molecule includes:
(a) a first polynucleotide having the nucleotide sequence shown in SEQ ID NO: 1 or SEQ ID NO: 2; and
(b) a second polynucleotide having the nucleotide sequence shown in SEQ ID NO: 3.
wherein (b) and (a) are 5'-3'linked, and said nucleic acid molecule is able to facilitate efficient transcription of the heterogeneous polynucleotide in liver tissue of mammal.

In some examples, the nucleic acid molecule also includes:
(c) a third polynucleotide having a nucleotide sequence shown in SEQ ID NO: 4, or a functional fragment of the third polynucleotide;
wherein (c), (b) and (a) are 5'-3' linked, and the nucleic acid molecule is able to facilitate efficient transcription of the heterogeneous polynucleotide in liver tissue of mammal.

In some other examples, the nucleic acid molecule includes:
(a) a first polynucleotide having the nucleotide sequence shown in SEQ ID NO: 1 or SEQ ID NO: 2; and
(b) a second polynucleotide having the nucleotide sequence shown in SEQ ID NO: 3; and
(c) a third polynucleotide having the nucleotide sequence shown in SEQ ID NO: 4;
wherein (c), (b) and (a) are 5'-3' linked, and the nucleic acid molecule is able to facilitate efficient transcription of the heterogeneous polynucleotide in liver tissue of mammal.

The present disclosure also provides an application of the nucleic acid molecule, and the nucleic acid molecule is used as a liver-specific promoter.

In some examples, the promoter can be used in but not limited to initiation of the expression of FVIII blood coagulation factor gene in liver.

The present disclosure also provides an expression vector including the nucleic acid molecule of the present disclosure, wherein the nucleic acid molecule is operably linked to the heterogeneous polynucleotide.

In preferred examples, the expression vector is a plasmid or a viral vector. Examples of the mammal expression vector include an adenovirus vector, plasmid vectors of pSV and pCMV series, a vaccinia vector and a retrovirus vector, as well as a baculovirus vector. In some embodiments, the expression vector is an adeno-associated virus vector rAAV.

The expression vector also includes one or more of the following elements: a replication origin, a selectable marker and a multiple cloning site.

The present disclosure also provides a mammal host cell including the expression vector of the present disclosure. The expression vector can be transfected into the host cell by any suitable method. Preferably, the host cell is a mammal cell (such as a human cell), for example those mentioned above. These host cells may be isolated cells.

The nucleic acid molecule is able to facilitate the transcription of the heterogeneous polynucleotides operably linked in the mammal cell. Preferred mammal cells include a mouse cell, a rat cell, a hamster cell, a monkey cell and a human cell. Examples of such cells include an HEK cell and a derivative (such as HEK293, HEK293T, HEK293A), a PerC6 cell, 911 cell, a CHO cell, an HCT116 cell, an HeLa cell, a COS cell and a VERO cell; cancer cells such as HepG2, A549 and MCF7; primary cells isolated from human or animal in biopsy; and stem cells (including pluripotent cells, such as embryonic stem cells and induced pluripotent stem cells (iPS); as well as pluripotent stem cells, such as hemopoietic stem cells, mesenchymal stem cells, and so forth).

A person skilled in the art will easily understand that, the promoter provided by the present disclosure can be used to not only initiate the expression of FVIII blood coagulation factor gene in liver, but also initiate the expression of non-encoded RNA, such as initiate the expression of siRNA.

The following are examples:

### Example 1

### Recombinant promoter fragment

Production of the recombinant promoter fragment consisting of the sequence identified in Table 1.

**Table 1: Sequence of the recombinant promoter fragment**

| Name of promoters | Third polynucleotide | Second polynucleotide | First polynucleotide |
|---|---|---|---|
| ATh1 promoter | None | SEQ ID NO: 3 | SEQ ID NO: 2 |
| ATh2 promoter | None | SEQ ID NO: 3 | SEQ ID NO: 1 |
| ATh3 promoter | SEQ ID NO: 4 | SEQ ID NO: 3 | SEQ ID NO: 2 |
| ATh4 promoter | SEQ ID NO: 4 | SEQ ID NO: 3 | SEQ ID NO: 1 |
| ATh5 promoter | SEQ ID NO: 5 | SEQ ID NO: 3 | SEQ ID NO: 2 |

The first polynucleotide, the second polynucleotide and the third polynucleotide (if present) are consecutively linked in the above-mentioned promoter fragment.

### Example 2

### Construction of the rAAV expression vector including different promoters

Human α1-antitrypsin (hAAT) is a serine protease inhibitor encoded and synthesized by SERPINA1 gene, it is primarily synthesized by liver and secreted into blood; an hAAT promoter, as a liver-specific promoter, has a stronger initiation capacity, its expression regulatory elements are dispersed within a range of 1.3Kb (-1200bp-+46bp) (Perlino et al., 1987; Rong-Fong Shen et al., 1989), the larger size restricts its application in the AAV vector. Literature reviews indicate that, an hAAT core promoter region is located in the -142/+44bp region of a transcription start site (TSS), in which transcription factor binding sites such as HNF1, HNF4, CEBP are distributed. In a remote enhancer region (Distal region, DRI), -261/-181bp upstream of the transcription start site (TSS), there also exists binding sites for transcription factors CEBP and HNF3, they regulate high level expression of the hAAT in liver tissue.

The present disclosure chose a -142/+44bp region of the hAAT promoter (186bp, NCBI Reference Sequence: NG_008290.1: 6948nt-7133nt), named as a mini-hAAT promoter; and chose a -261/+44bp region of the hAAT promoter (305bp, NCBI Reference Sequence: NG_008290.1: 6829nt-7133nt), named as an hAAT promoter; and chose a -142/-62bp region of the hAAT promoter (81bp, NCBI Reference Sequence: NG_008290.1: 6948nt-7029nt, SEQ ID NO: 3), named as a truncated-hAAT hAAT promoter; and chose a - 261/-208bp region of the hAAT promoter (54bp, NCBI Reference Sequence: NG_008290.1: 6829nt-6882nt, SEQ ID NO: 4), named as hAAT enhancer/hAATe.

Transthyretin (TTR) is a thyroxine transporter, it is secreted into blood after being primarily synthesized in liver tissue, and it is secreted into cerebrospinal fluid after being synthesized in choroid plexus epidermic cells. Expression of mouse TTR is regulated by two liver-specific regions: a proximal regulatory region of - 200/+1 (a promoter region) and a distal regulatory region of -1.86/-1.96 kbp (an enhancer region). A TTR promoter, as a liver-specific promoter, due to its smaller size and stronger initiation effects, is also widely used in gene therapy. Multiple transcription factor binding sites such as AP1, CEBP, HNF4, HNF1, HNF3 are distributed in mouse TTR enhancer and promoter regions, they regulate the efficient and specific expression of TTR protein.

The present disclosure obtains multiple chimeric promoters by chimerism of important regulatory regions of the hAAT promoter and the mouse TTR promoter; five promoters with stronger initiation effects being screened out in subsequent tests are respectively named as an ATh1 promoter, an ATh2 promoter, an ATh3 promoter, an ATh4 promoter and an ATh5 promoter.

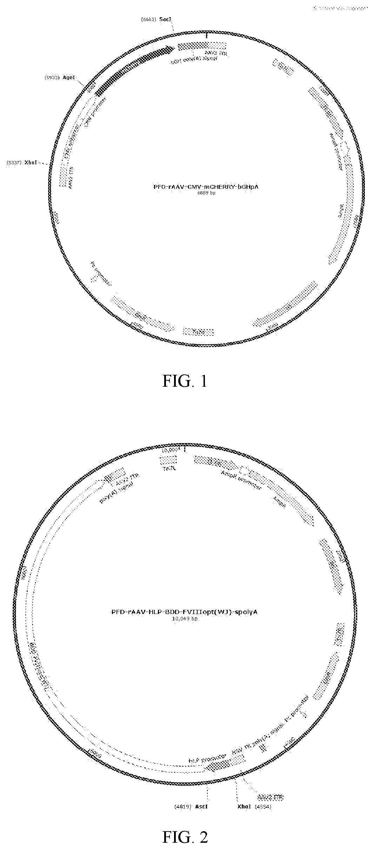

To compare the initiation intensities of different promoters, the present disclosure constructs the above-mentioned chimeric promoters and the above-mentioned stronger liver-specific promoter that has been reported together onto PFD-rAAV-CMV-mCHERRY- bGHpA of the AAV vector, as shown in FIG 1. Based on the initiation of mCHERRY fluorescent protein expression, initiation levels of different promoters are compared. In FIG. 1, ITR (inverted terminal repeat) is an inverted terminal repeat with a length of 145bp; CMV enhancer/promoter is human cytomegalovirus early promoter; mCHERRY is red luciferase gene reading frame; BGH polyA is bovine growth hormone polyadenylation signal; Amp is ampicillin resistant gene reading frame; GmR is hygromycin resistant gene reading frame; Tn7F/R is Tn7 transposon; ori is replication origin site; XhoI, AgeI, SacI are restriction endonuclease sites. The detailed construction strategy is:
1) PFD-rAAV-CMV-mCHERRY-bGHpA is double digested with restriction enzymes XhoI and AgeI, and linearized;
2) a primer is designed for synthesis of a promoter fragment required for template amplification, when the primer is designed, a restriction enzyme site and a homologous recombinant arm of approximately 18bp are reserved for recombination with the vector after the linearization;
3) ClonExpress II One Step Cloning Kit (Vazyme, C112) and ClonExpress MultiS One Step Cloning Kit (Vazyme, C113) are chosen on the basis of the number of the needed recombinant fragments to perform a homologous recombination on the target fragment and the vector backbone; and
4) the homologous recombination product is transformed into STBL3 competence, coated onto a LB plate and cultivated at 37°C overnight and the monoclone is picked for identification, the clone identified correctly is shaken and plasmid is extracted.

The detailed sequence information is as follow:
hAAT promoter (-261/+44bp): (NCBI Reference Sequence: NG_008290.1: 6829nt-7133nt)
mini-hAAT promoter (-142/+44bp): (NCBI Reference Sequence: NG_008290.1: 6948nt-7133nt)
mTTR promoter (NCBI Reference Sequence: NC_000084.6, 1961nt-2164nt,-203/+1bp), SEQ ID NO: 1
mini-mTTR promoter (NCBI Reference Sequence: NC_000084.6, 2014nt-2164nt, -150/+1bp), SEQ ID NO: 2
truncated-hAAT hAAT promoter (-142/-62bp): (NCBI Reference Sequence: NG_008290.1: 6948nt-7029nt), SEQ ID NO: 3
AAT enhancer (NCBI Reference Sequence: NG_008290.1: 6829nt-6882nt), SEQ ID NO: 4
mTTR enhancer (NCBI Reference Sequence: NC_000084.6, 286nt-405nt, -1878/-1758bp), SEQ ID NO: 5
ATh1 Promoter, SEQ ID NO: 6
ATh2 Promoter, SEQ ID NO: 7
ATh3 Promoter, SEQ ID NO: 8
ATh4 Promoter, SEQ ID NO: 9
ATh5 Promoter, SEQ ID NO: 10
Biomarin-HLP promoter, promoter in BMN270 project in BIOMARIN Company, ClinicalTrials.gov number, NCT02576795
SPARK-TTRm (NCBI Reference Sequence: NC_000084.6, 1961nt-2183nt, -203bp/+20bp), wherein - 136bp/-133bp TGTG mutation is GACT
ApoE enhancer (GenBank: U32510.1, 78nt-231nt)
HS-CRM8 enhancer (NCBI Reference Sequence: NM_000295.4, 163 nt- 93nt)
MVM intron (GenBank: NC_001510.1, 2312nt-2403nt)

### Example 3

### Comparison of effects of different promoters for initiating mCHERRY fluorescent gene

The promoter, fluorescent protein mCHERRY and bGHpolyA AAV expression vector constructed in Example 2 were packaged into the AAV2/8 serotype virus through a 293 three-plasmid system, and the viruses were precipitated with PEG8000, then AAV virus was purified by iodixanol density gradient ultracentrifugation. Virus titer was quantified by RT-PCR and silver staining.

Different AAV viruses were injected into bodies of C57 mice (n=3) at the same dose of 3E+13vg/kg by tail vein injection, 5 weeks after injection of the viruses, heart perfusion was conducted with physiological saline and 4% paraformaldehyde, and the heart, liver, spleen, lung, kidney, brain etc. were taken and immersed in 4% paraformaldehyde overnight, and then immersed in a 30% sucrose solution for 72 hours. Liver tissue was taken and low temperature freezing microtomy was conducted, and fluorescence was observed under a fluorescence microscope.

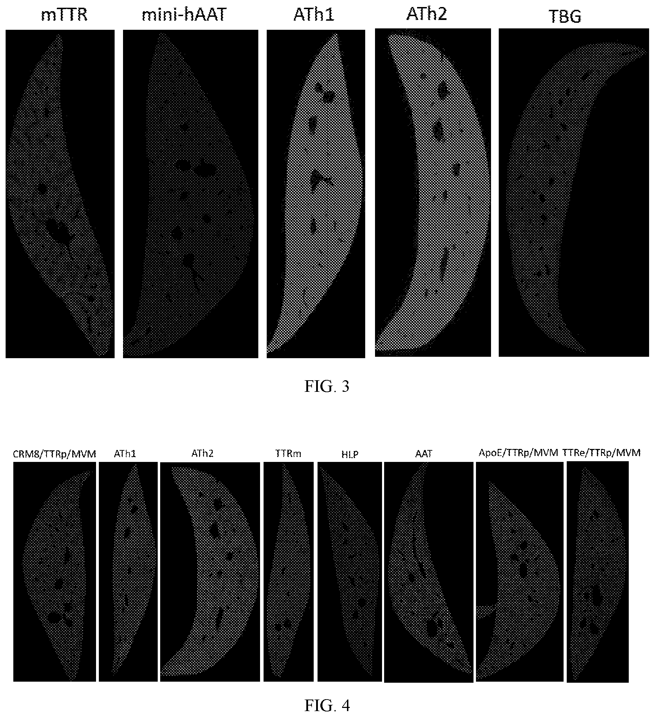

A comparison of mCHERRY fluorescence was conducted between the ATh1 Promoter (SEQ ID NO: 6) and ATh2 Promoter (SEQ ID NO: 7) after the recombination of the present disclosure and the mini-hAAT promoter, mTTR promoter (SEQ ID NO: 1, a mouse TTR promoter region), TBG promoter, hAAT promoter, TTRm promoter (TTR promoter after mutation in spark-8011 project in SPARK Company, ClinicalTrials.gov number, NCT03003533), HLP promoter (promoter in BMN270 project in BIOMARIN Company, ClinicalTrials.gov number, NCT02576795), TTRe/TTRp promoter (a mouse TTR enhancer region and a promoter region), TTRe/TTRp/MVM (MVM intron that was added behind the mouse TTR enhancer region and promoter region) and CRM8//TTRp/MVM (MVM intron that was added behind the mouse TTR promoter region, CRM8 enhancer sequence that was added in front of the promoter region, promoter in SB525 project in sangoma Company, ClinicalTrials.gov number, NCT03061201), to compare their initiation levels in liver tissue, the results are as shown in FIG. **3** and FIG. **4****.**

FIG. **3** shows a comparison of effects of different promoters for initiating mCHERRY fluorescence in mouse liver tissue. AAV viruses of different promoters were injected into C57 mice (n=3) at the same dose of 3E+13vg/kg by tail vein injection, 5 weeks after injection of the viruses, liver tissue mCHERRY fluorescence intensities were compared. Exposure time was 10 ms left 20 and right 500.

FIG. **4** shows a comparison of effects of different promoters for initiating mCHERRY fluorescence in mouse liver tissue. AAV viruses of different promoters were injected into C57 mice (n=3) at the same dose of 3E+13vg/kg by tail vein injection, 5 weeks after injection of the viruses, liver tissue mCHERRY fluorescence intensities were compared. Exposure time was 10ms left 20 and right 4000.

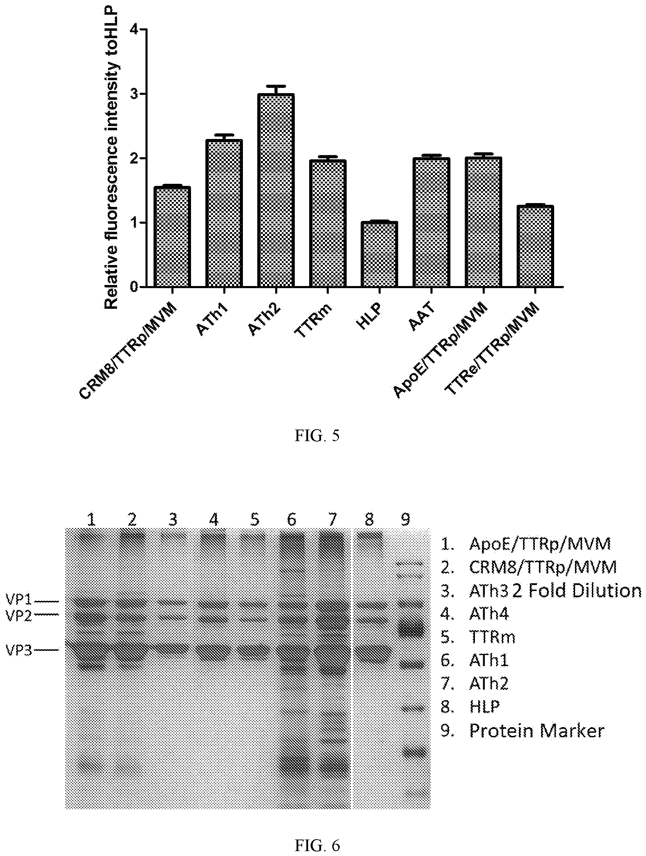

FIG. **5** shows a grayscale scan diagram of effects of different promoters for initiating mCHERRY fluorescence in mouse liver tissue. A grayscale scan was conducted for the mCHERRY fluorescence expressed by different promoters in mouse liver tissue in FIG. 4 by using graphpad software, the gray scale value was a relative intensity with respect to the HLP promoter.

By comparison of the mCHERRY fluorescences, the present disclosure finds that, in liver tissue, after the recombination, the initiation levels of promoters ATh1 Promoter (SEQ ID NO: 1) and ATh2 Promoter (SEQ ID NO: 2) were significantly superior to mini-hAAT promoter and mTTR promoter before recombination, and also superior to the TBG promoter, hAAT promoter, TTRm promoter, HLP promoter, TTRe/TTRp promoter, TTRe/TTRp/MVM and CRM8//TTRp/MVM.

### Example 4

### Construction of different promoters that initiate BDD-FVIII vector

To further discuss whether the strong liver-specific initiation levels of ATh1 and ATh2 promoters were influenced by the gene linked at downstream, as well as whether addition of expression regulatory enhancer elements (ATh3, ATh4) in front of ATh1 and ATh2 was able to further increase expression of the target gene; meanwhile to study whether ATh1-4 initiate expression of a large exogenous gene, and whether AAV was able to be normally packaged, this example constructed different promoters, BDD-FVIII (B domain deleted FVIII) and an sPolyA expression cassette according to the molecular cloning method in the above-mentioned Example 2, and they were constructed onto the AAV vector PFD-rAAV-HLP-BDD-FVIII-spolyA (FIG. 2), wherein the PFD-rAAV-HLP-BDD-FVIII-spolyA vector was double enzyme digested by using restriction enzymes XhoI and AscI, and linearized.

FIG. **2** shows a structural schematic diagram of PFD-rAAV-HLP-BDD-FVIIIopt (WJ)-spolyA vector. Wherein ITR (inverted terminal repeat) is a inverted terminal repeat with a length of 145bp; HLP promoter is a promoter (SEQ ID NO: 8) adopted for the project BMN270 of Biomarin Company for therapy of hemophilia A, BDD-FVIIIopt (WJ) is BDD-FVIII in which codon has been optimized; polyA is polyadenylation signal; Amp is ampicillin resistant gene reading frame; GmR is hygromycin resistant gene reading frame; Tn7F/R is Tn7 transposon; ori is replication origin site; XhoI and AscI are restriction enzyme sites.

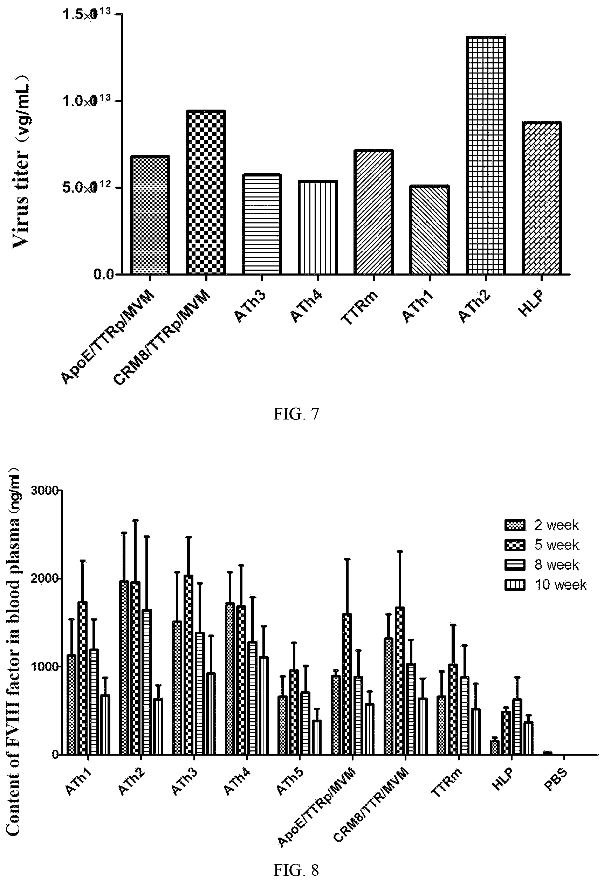

FIG. **6** shows SDS-PAGE protein gel silver staining diagrams for differentAAV viruses. The AAV viruses loaded with different promoters and FVIII gene expression cassettes were subjected to polyacrylamide gel electrophoresis, then a silver staining was conducted, to determine the purity and titer of the viruses. FIG. **7** shows different AAV virus titers determined by an RT-PCR method. Absolute quantitative PCR was conducted on AAV viruses in which different promoters express FVIII by using primers on the ITR, to determine the virus titer. From the silver staining result and the RT-PCR quantitative result, the above-mentioned vectors were able to be normally packaged.

### Example 5

### Comparison of levels of expressing FVIII by different promoters

The plasmid constructed in the above-mentioned Example 4 was packaged as the AAV2/8 serotype virus by the sf9 One-bac system. Different AAV viruses were injected into bodies of C57 mice (n=6) at the same dose of 5E+12vg/kg via tail veins, 2, 5, 8, 10 weeks after the virus injection, blood was sampled from orbital venous plexuses and the FVIII expression level was detected by ELISA. The ELISA antibody pair used was F8C-EIA (enzyme research laboratories), a standard curve was drawn by using human coagulation factor VIII (green cross), and the concentration of blood coagulation factor VIII was determined by using BiYunTian Enhanced BCA Protein Assay Kit (product number: P0010S).

FIG. **8** shows a comparison of the effects of different promoters for expressing FVIII in C57 mice. AAV viruses of different promoters were injected into C57 mice (n=6) at the same dose of 5E+12vg/kg by tail vein injection, 2 weeks, 5 weeks, 8 weeks, 10 weeks after injection of the viruses, mouse blood plasma were collected respectively, and the FVIII content in blood plasma was determined by an ELISA method.

From the overall trend of the detection results, due to timeliness of the AAV expression, from week 2 to week 5, the levels of promoters for expressing FVIII all increased, at week 5 the expression level reached the highest level, at the subsequent two time-points, the expression levels all decreased by different degrees.

As a whole, at each time-point, the capacity of initiating FVIII by ATh1, ATh2, ATh3, ATh4 are significantly superior to TTRm of Spark Company and HLP promoter of Biomarin Company, at week 2, the levels of expressing FVIII by these four promoters were 2-3 times of that of TTRm, and 10 times or above of that of HLP, at week 5, the levels of expressing FVIII by these four promoters were 1.5-2 times of that of TTRm, and 3-4 times of that of HLP, at week 8, the levels of expressing FVIII by these four promoters were 1.3-1.9 times of that of TTRm, and 1.9-2.6 times of that of HLP, and at week 10, the levels of expressing FVIII by these for promoters were 1.2-2.1 times of that of TTRm, and 1.7-3 times of that of HLP; compared to ApoE/TTRp/MVM and CRM8/TTRp/MVM promoters, at each time-point, levels of expressing FVIII by these four promoters also increased by 1-2 times, although the increasing effect was not evident compared to those of mTTR and HLP, sizes of ATh1 and ATh2 reduced by 83-21 1bp, this is important for AAV with limited packaging capacity to load macromolecules such as FVIII. Compared with ATh1 and ATh2, the expression levels of ATh3 and ATh4 increased by 1.4-1.8 times at week 10, although the effect on expression did not significantly increase in the early by adding some expression enhancer elements, being more superior in respect of expression persistence.

However, for the recombinant promoter ATh5 using the same strategic component, its effect of expressing FVIII is significantly less than those of the other four recombinant promoters proposed by the present disclosure, indicating that there is not necessarily a synergistic effect on the chimerism between the different expression regulatory elements, and there might also be a situation of competing for transcription factors; based on analysis, on one hand, there may be a steric effect within a small space range, binding of one transcription factor prevents other transcription factor from entering an adjacent site, on the other hand, there is certain interaction between different transcription regulatory factors, not just a sequential arrangement, and the additionally bonded regulatory factors may disrupt the original interaction.

The base numbers of the constructed recombinant promoters and other expression regulatory elements for comparison of the present disclosure are listed in Table 2.

**Table 2: Base number of different expression regulatory elements**

| Name | Base number (bp) |
|---|---|
| hAAT promoter (-261/+44bp) | 305 |
| mini-hAAT promoter (-142/+44bp) | 186 |
| hAAT enhancer (-261/-208bp) | 54 |
| Truncted-hAAT promoter (-142/-62bp) | 81 |
| mTTR enhancer | 99 |
| mTTR promoter | 203 |
| mini-mTTR promoter | 151 |
| TBG promoter | 460 |
| ATh1 promoter | 232 |
| ATh2 promoter | 284 |
| ATh3 promoter | 286 |
| ATh4 promoter | 338 |
| ATh5 promoter | 331 |
| Biomarin-HLP promoter | 252 |
| SPARK-TTRm | 223 |
| ApoE enhancer | 154 |
| HS-CRM8 enhancer | 78 |
| MVM intron | 92 |
| CRM8/TTRp/MVM | 366 |
| ApoE/TTRp/MVM | 449 |
| TTRe/TTRp/MVM | 394 |

As known from the above table, the recombinant promoters of the ATh1 promoter and ATh2 promote provided by the present disclosure achieve an effect of increasing the FVIII expression level by 1.2-10 times at a size similar to HLP and TTRm (ATh1, 232bp; ATh2, 284bp; HLP, 252bp; TTRm, 223bp), compared with ApoE/TTRp/MVM and CRM8/TTRp/MVM promoter, the level of expressing FVIII also increases by 1-2 times, whereas the size reduces by 83-21 1bp. For ATh3 and ATh4 (ATh3, 286bp; ATh4, 338bp), compared with ATh1 and ATh2, their lengths slightly increase, but time duration for efficiently expressing FVIII is longer.

## Claims

1. A nucleic acid molecule, wherein the nucleic acid molecule comprises:
(a) a first polynucleotide having a nucleotide sequence shown in SEQ ID NO. 1 or SEQ ID NO. 2; and
(b) a second polynucleotide having a nucleotide sequence shown in SEQ ID NO. 3;
wherein the second polynucleotide (b) and the first polynucleotide (a) are 5'-3'linked, and the nucleic acid molecule is able to facilitate transcription of a heterogeneous polynucleotide in liver tissue of mammal.

2. The nucleic acid molecule according to claim 1, wherein the nucleic acid molecule also comprises:
(c) a third polynucleotide having a nucleotide sequence shown in SEQ ID NO. 4;
wherein (c), (b), and (a) are 5'-3'linked, and the nucleic acid molecule is able to facilitate transcription of the heterogeneous polynucleotide in liver tissue of mammal.

3. An expression vector, wherein the expression vector comprises the nucleic acid molecule according to any one of claims 1 to 2.

4. The expression vector according to claim 3, wherein the expression vector is a plasmid or a viral vector.

5. A mammal host cell, comprising the expression vector according to claim 3 or 4.

## Patentansprüche

1. Nukleinsäuremolekül, wobei das Nukleinsäuremolekül Folgendes umfasst:
(a) ein erstes Polynukleotid mit einer Nukleotidsequenz, dargestellt in SEQ ID NO. 1 oder SEQ ID NO. 2, und
(b) ein zweites Polynukleotid mit einer Nukleotidsequenz, dargestellt in SEQ ID NO. 3,
wobei das zweite Polynukleotid (b) und das erste Polynukleotid (a) 5'-3'-verknüpft sind und das Nukleinukleinsäuremolekül in der Lage ist, die Transkription eines heterogenen Polynukleotids in Lebergewebe von Säugetieren zu erleichtern.

2. Nukleinsäuremolekül nach Anspruch 1, wobei das Nukleinsäuremolekül auch Folgendes umfasst:
(c) ein drittes Polynukleotid mit einer Nukleotidsequenz, dargestellt in SEQ ID NO. 4,
wobei (c), (b) und (a) 5'-3'-verknüpft sind und das Nukleinsäuremolekül in der Lage ist, die Transkription des heterogenen Polynukleotids in Lebergewebe von Säugetieren zu erleichtern.

3. Expressionsvektor, wobei der Expressionsvektor das Nukleinsäuremolekül nach einem der Ansprüche 1 bis 2 umfasst.

4. Expressionsvektor nach Anspruch 3, wobei der Expressionsvektor ein Plasmid oder ein viraler Vektor ist.

5. Säugetier-Wirtszelle, umfassend den Expressionsvektor nach Anspruch 3 oder

## Revendications

1. Molécule d'acide nucléique, dans laquelle la molécule d'acide nucléique comprend :
(a) un premier polynucléotide présentant une séquence nucléotidique représentée dans SEQ ID NO. 1 ou SEQ ID NO. 2 ; et
(b) un deuxième polynucléotide présentant une séquence nucléotidique représentée dans SEQ ID NO. 3 ;
dans laquelle le deuxième polynucléotide (b) et le premier polynucléotide (a) sont liés en 5'-3', et la molécule d'acide nucléique est capable de faciliter la transcription d'un polynucléotide hétérogène dans le tissu hépatique de mammifère.

2. Molécule d'acide nucléique selon la revendication 1, dans laquelle la molécule d'acide nucléique comprend également :
(c) un troisième polynucléotide présentant une séquence nucléotidique représentée dans SEQ ID NO. 4 ;
dans laquelle (c), (b) et (a) sont liés en 5'-3', et la molécule d'acide nucléique est capable de faciliter la transcription du polynucléotide hétérogène dans le tissu hépatique de mammifère.

3. Vecteur d'expression, dans lequel le vecteur d'expression comprend la molécule d'acide nucléique selon l'une quelconque des revendications 1 à 2.

4. Vecteur d'expression selon la revendication 3, dans lequel le vecteur d'expression est un plasmide ou un vecteur viral.

5. Cellule hôte de mammifère, comprenant le vecteur d'expression selon la revendication 3 ou 4.
